Europäisches Patentamt

European Patent Office

Office européen des brevets

(19)

(11) Publication number: **0 410 228 B1**

# EUROPEAN PATENT SPECIFICATION

(12)

(45) Date of publication of patent specification: **15.06.94**

(51) Int. Cl.5: **C12N 15/75**, //(C12N1/21, C12R1:125)

(21) Application number: **90113416.3**

(22) Date of filing: **13.07.90**

(54) **The thermo-inducible expression of heterologous genes in bacillus subtilis and means and methods for its achievement.**

(30) Priority: **27.07.89 IT 2134389**

(43) Date of publication of application:
**30.01.91 Bulletin 91/05**

(45) Publication of the grant of the patent:
**15.06.94 Bulletin 94/24**

(84) Designated Contracting States:
**AT BE CH DE DK ES FR GB GR LI LU NL SE**

(56) References cited:
**EP-A- 0 182 562**
**DE-A- 3 619 902**

**CHEMICAL ABSTRACTS, vol. 110, no. 9, 27th February 1989, page 173, abstract no.70450k, Columbus, Ohio, US; A.V. SOROKIN et al.: "Use of lambda phage promoters for the expression of secreted protein genes in Bacillus subtilis cells"**

(73) Proprietor: **ENIRICERCHE S.p.A.**
**Corso Venezia 16**
**I-20121 Milan(IT)**

(72) Inventor: **Cosmina, Paola**
**Viale Misurata, 14**
**I-20146 Milano(IT)**
Inventor: **Velati Bellini, Ada**
**Via Libertà, 17/1**
**I-27100 Pavia(IT)**
Inventor: **De Ferra, Francesca**
**Via Europa, 30**
**I-20097 San Donato Milanese, Milan(IT)**
Inventor: **Grandi, Guido**
**Nona Strada, 4**
**I-20090 Segrate, San Felice, Milan(IT)**

(74) Representative: **Perani, Aurelio et al**
**c/o JACOBACCI-CASETTA & PERANI S.p.A**
**7, Via Visconti di Modrone**
**I-20122 Milano (IT)**

EP 0 410 228 B1

GENE, vol. 5, 1979, pages 59-76, Amsterdam, NL; H.-U. BERNARD et al.:"Construction of plasmid cloning vehicles that promote gene expression from the bacteriophage lambdapL promoter"

APPLIED MICROBIOLOGY AND BIOTECHNOLOGY, vol. 24, 1986, pages 504-508, Berlin, DE; Y. MUROOKA et al.: "Utilization of promoter regions of coliphage lambda in Bacillus subtilis"

## Description

The present invention generally relates to the regulation of the expression of heterologous genes in Bacillus subtilis (B.subtilis) by temperature variation and to means and methods for its achievement.

In particular, the present invention relates to a plasmid vector which can be replicated in B.subtilis and which is useful for the thermo-inducible expression and cloning of a heterologous gene which codes for a protein of interest, in which the gene is put under the control of a strong promoter whose activity is modulated by the heat-sensitive repressor of the lambda phage of E.coli by means of a variation in temperature.

It is known that the level of expression of a heterologous structural gene in a microorganism depends strictly on the strength of its promoter, that is, on its capacity to give rise to messenger RNA at a high frequency. Often, however, a high level of transciption may, on the one hand, give rise to a gene product which, precisely because it is expressed in large quantities, may be toxic to the cell and, on the other hand, may interfere with the mechanisms for the replication of the DNA and lead to plasmid instability (Remaut et al., Gene, 15, 18, 1981).

There is therefore a need for promoters whose activity can be modulated by means of an induction/repression system connected thereto. The expression of a gene controlled by such a promoter can thus be achieved by means of an external chemical or physical modulator which enables the exact moment at which expression is started to be selected.

The activity of some promoters can be modulated by a variation in temperature. The expression of the gene controlled by such promoters thus starts only at a certain temperature.

Many systems for the regulation of genes in gram-negative bacteria, such as strains of Escherichia coli, are known from technical and patent literature.

Examples are the trp promoter, which is repressed by tryptophan and induced by 3-indolyl acetic acid (Morse et al., 1970, Cold Spring Harbor Symp. Quant. Biol., 34:725), the lac promoter, which is regulated by the lac repressor and induced by IPTG (isopropyl thiogalactopyranoside) and the $P_L$ and $P_R$ promotors of the lambda phage of E. coli. In particular, the $P_L$ promoter, which is a well-regulated, strong promoter, has been used for the construction of vectors with expression in E.coli (Hedgpeth et al., Mol. Gen. Genet., 163: 197, 1981; Bernard et al., Gene, 5: 59, 1979; Shimatake and Rosemberg, Nature, 292: 128, 1981).

The regulation of this promoter is achieved only if the gene clts 857, which codes for a heat-sensitive protein (repressor) with 236 aminoacids (MW 27000) is present in the same vector or on the bacterial chromosome (Bernard et al., Gene, 5, 59, 1979). In the lambda phage, at low temperature (28°C), this repressor binds by means of its aminoterminal portion to the sequences known as the "operators ($O_L$)" situated within the $P_L$ promoter, keeping the promoter itself in a repressed, that is inactive, state. If the temperature is increased (up to 42°C), the activity of the repressor is eliminated and $P_L$ is activated.

A similar activation mechanism is observed with the $P_R$ promoter (Lambda II, ed. Hendrix, Roberts, Stahland Weisberg (CSM Laboratory, New York 1983)).

As regards B.subtilis, although it possessess regulatory systems which are complex and as yet unclear, up to now, none of these has shown the characteristics required in a regulatory system which can be used for the development of expression vectors, that is:

a) very low levels of expression of the gene in the repressed state: this appears to be particularly important when the gene produced is toxic to the host cell;

b) high levels of expression in the non-repressed state: once induced, the gene must be activated and levels of expression of the gene more than one thousand times higher than the levels found in the represssed stated should be reached.

There is therefore a need for inducible systems which are effective in B.subtilis and, in particular, for thermo-inducible systems which enable simple and economically convenient methods to be established.

Surprisingly, it has now been found that the expression of a heterologous gene in B.subtilis (gram-positive) can be regulated by the construction of a plasmid vector according to the present invention which uses the inducible system of the lambda phage of E.coli (gram-negative).

A subject of the present invention is therefore constituted by a plasmid vector replicable in B.subtilis, which is useful for the cloning and thermo-inducible expression of a heterologous structural gene which codes for a protein of interest, in which the gene is put under the control of a strong promoter whose activity is modulated, by means of temperature variation, by the heat-sensitive repressor of the lambda phage of E.coli.

Another subject of the present invention is a method for the thermo-inducible expression in B.subtilis of a heterologous gene which codes for a protein of interest, comprising the growth of a strain of B.subtilis transformed by the plasmid vector in a suitable culture medium and at a temperature which may vary from 28°C to 42°C.

Further subjects of the present invention will become clear from a reading of the description and the examples which follow.

In particular, the inducible system used for the construction of the plasmid vector according to the present invention is constituted by the structural gene cIts857, which codes for a heat-sensitive repressor protein, and by its operator/promoter $O_L/P_L$.

The system can be introduced into a vector replicable with expression in B.subtilis selected from those known in the art.

Preferably the plasmid pSM 214 ATCC 67320, a vector which is functional both in E.coli and B.subtilis, whose construction is described in EP-A-0281530, is used. This plasmid is characterised in that it contains the gene which codes for resistance to kanamycin and a promoter responsible for the synthesis of a dicystronic mRNA which includes the sequences of the Bla and Cat structural genes. The removal of the Bla gene by means of the restriction enzymes EcoRI and HindIII and the subsequent insertion of a heterologous nucleotide sequence in the sites enables plasmids to be constructed in which the transcription of the sequence is ensured by the presence of the single promoter.

In practice, a 710 bp DNA fragment corresponding to the structural gene which codes for the repressor protein was isolated from the lambda phage of E.coli (GIBCO-BRL) and inserted in the plasmid pSM 214 ATCC 67320 in the single restriction sites of EcoRI and HindIII to produce the plasmid pSM 312. The construction was carried out by techniques generally known in the field of recombinant DNA. In order to check whether the protein was expressed by B.subtilis which is a gram-positive microorganism, cells transformed by the vector were grown on a suitable culture medium to which chloramphenicol had been added, both at 28°C and at 42°C.

The total protein content extracted from the proteinaceous lysate was analysed on polyacrylamide gel and showed the presence of a protein with a molecular weight corresponding to that of the repressor. This confirmed the capacity of B.subtilis to express the heterologous protein.

A synthetic oligonucleotide sequence containing the operator $O_L$ was then inserted in the plasmid pSM 312 upstream of the structural gene cIts 857, the plasmid pSM 312$O_L$ was isolated and, upon restriction analysis, was shown to have the correct construction.

E.coli and B. subtilis cells transformed by the plasmid, grown both in a liquid medium and on plates with the addition of chloramphenicol, were capable of expressing chloramphenicol acetyl transferase only at 42°C. This indicated that the thermo-inducible system used was also able to function in B.subtilis.

The plasmid thus appeared to be particularly suitable for the thermo-inducible expression of heterologous genes by the replacement of the Cat gene by a gene which codes for a different protein.

The plasmid pSM 312$O_L$ was deposited at the American Type Culture Centre as B.subtilis SMS 118 (pSM 312$O_L$) ATCC 68058.

## BRIEF DESCRIPTION OF THE DRAWINGS

Figure 1: the construction of the plasmid pSM 312 is shown schematically.

Figure 2: 12.5% SDS polyacrylamide gel of the total protein content extracted from the cell lysate of E.coli and B.subtilis cells grown at 42°C.

Figure 3: the construction of the plasmid pSM 312$O_L$ is shown schematically.

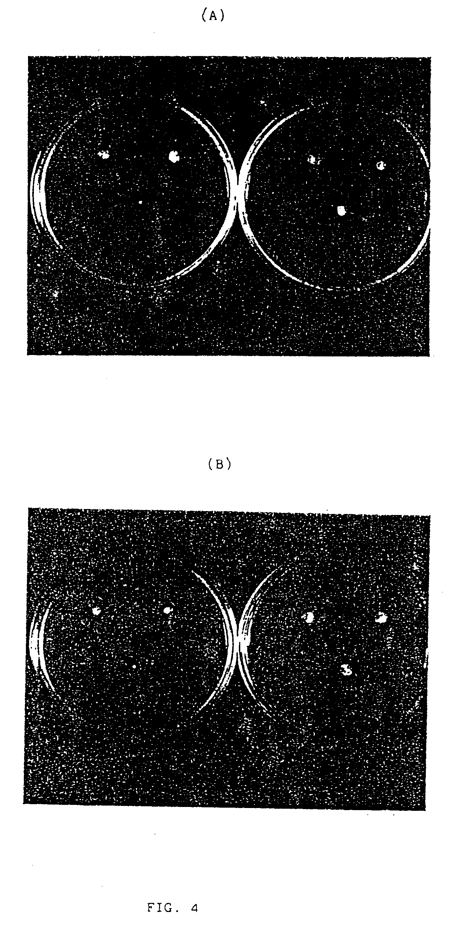

Figure 4 (A and B): the culture of E.coli and B.subtilis cells grown on plates at 28°C and 42°C is shown.

The following experimental examples are illustrative of the invention and are not limiting.

## Example 1

### Isolation of the structural gene cIts 857 from the DNA of the lambda phage

0.5μg of the DNA of the lambda phage (GIBCO BRL) were digested with 2 units (U) of the restriction enzyme EcoRI (BRL) in 20μl of a buffer solution containing 50mM NaCl, 100mM pH7.5 Tris-HCl, and 5mM $MgCl_2$, for 1 hour at 37°C. The plasmid DNA, thus linearised, was extracted with equal volumes of phenol-chloroform/chloroform-isoamyl alcohol, precipitated with 0.3M final of sodium acetate and 2.5 volumes of ethanol for 10 minutes at -80°C and finally centrifuged in an Eppendorf centrifuge, model 5450, for 10 minutes at 12,000 rpm at 4°C. The DNA precipitated was isolated and dried under vacuum.

The 710bp structural gene cIts 857 was then isolated from the plasmid DNA by the enzymatic amplification method with the aid of a primer with a heat-stable DNA polymerase (Tag polymerase) (Erlich et al., Science, 239: 487-491, 1988).

In practice, two complementary oligonucleotides were synthesised each at the 3′ end of one of the two helices of the gene and each containing an EcoRI restriction site immediately adjacent the ATG which starts the translation of the gene to facilitate cloning in the pSM 214 vector.

The two oligonucleotides have the following sequences (SED. ID. No. 1 and 2):

```
                          Eco RI
        L1 = 5'GTGAATTCTTATGAGCACAAAAAAGAAACCA 3'
        L2 = 5'GTAAGCTTTCAGCCAAACGTCTCTTCAGG 3'
```

30 ng of the phage DNA, linearised and separated as described above, were amplified by addition to 100 picomoles (1μl each) of the two oligonucleotides suspended in water, 200μM of each deoxynucleotide (dATP, dGTP, dCTP, dTTP), 200μg/ml of BSA (Boehringer), 50mM KCl, 10mM pH8.4 Tris-HCl, 2.5mM MgCl$_2$ and 2U of the heat-stable enzyme Taq polymerase (Biolabs). The sample was covered with paraffin to prevent its evaporation and was then subjected to 30 reaction cycles, each constituted as follows:
- 1 minute at 95°C to denature the DNA;
- 1 minute at 40°C to promote the annealing of the oligonucleotides to the individual helices;
- 5 minutes at 70°C to activate the amplification operation achieved by the polymerase.

5 μl samples were taken after the 10th and 20th cycles, examined on agarose gel and showed an increase in the DNA due to its amplification. The reaction was stopped by extraction with an equal volume of phenol-chloroform/chloroform-isoamyl alcohol and the DNA precipitated with 0.3M sodium acetate and 2.5 volumes of ethanol. A portion of the precipitate, examined on agarose gel enabled the 710bp DNA fragment recovered to be evaluated at a total of 5μg.

2μg of this fragment were digested in 20μl of a buffer containing 50mM NaCl, 100mM pH7.5 Tris-HCl, 5mM MgCl$_2$ with 5U of EcoRI at 37°C for 1 hour in order to make its ends cohesive. The reaction was stopped and the DNA precipitated as described above. The DNA, separated by centrifuging, was then resuspended in 20 μl of water for use in the subsequent ligation reaction.

Example 2

Construction of the plasmid pSM 312.

In order to confirm the ability of B. subtilis to express the repressor, the gene which codes for the repressor was cloned in the vector pSM 214 ATCC 67320 with expression in B.subtilis and in E.coli, which vector contains the structural genes which code for resistance to ampicillin (Bla) and to chloramphenicol (Cat) and which are put under the control of a promoter responsible for the synthesis of a dicystronic mRNA.

a) Insertion of the structural gene cIts 857 in pSM 214

1μg of the plasmid pSM 214 ATCC 67320 was digested with 2U of the restriction enzyme HindIII (Boehringer) in 20μl of a buffer containing 50mM NaCl, 50mM of pH7.5 Tris-Hcl and 10mM of MgCl$_2$ for 1 hour at 37°C. After the reaction had been stopped and the DNA precipitated as described above, this was suspended in 25μl of a buffer containing 50mM pH7.2 Tris-Hcl, 10mM MgSO$_4$, 0.1mM dithiothreitol (DTT), 50gamma/ml BSA, 80μM (final concentration) of each of the triphosphate deoxynucleotides (dATP, dGTP, dCTP, dTTP) and 2U of Klenow DNA polymerase enzyme (Boehringer). The resulting mixture was kept at 23°C for 30 minutes and the reaction was then stopped by treatment with equal volumes of phenol-chloroform/chloroform-isoamyl alcohol. The DNA was precipitated as described above, resuspended in 20μl of a buffer containing 50mM NaCl, 500mM pH7.5 Tris-HCl, 5mM MgCl$_2$ and 2U EcoRI and kept at 37°C for 1 hour. After the enzymatic reaction had been stopped, the DNA was precipitated as described above and the 6520 bp DNA fragment, without the beta-lactamase structural gene (Bla), was isolated.

The DNA fragment and 300ng of the 710bp DNA fragment were resuspended in 100μl of ligation mixture (50mM pH7.6 Tris-HCl, 10mM MgCl$_2$, 5mM DTT, 0.1mM spermidine, 0.1mM EDTA, 1mM ATP) and reacted at 14°C for 16 hours in the presence of 1U of T4 DNA ligase (Boehringer). After the reaction had been stopped at 65°C for 10 minutes, the entire ligation mixture was used to transform E.coli 71.18 cells (lac pro SupE thi F′ proAB LacIq Z.. M15) made competent by the method described by Messing (Methods Enzymology Vol. 101, 20-78 (1983)).

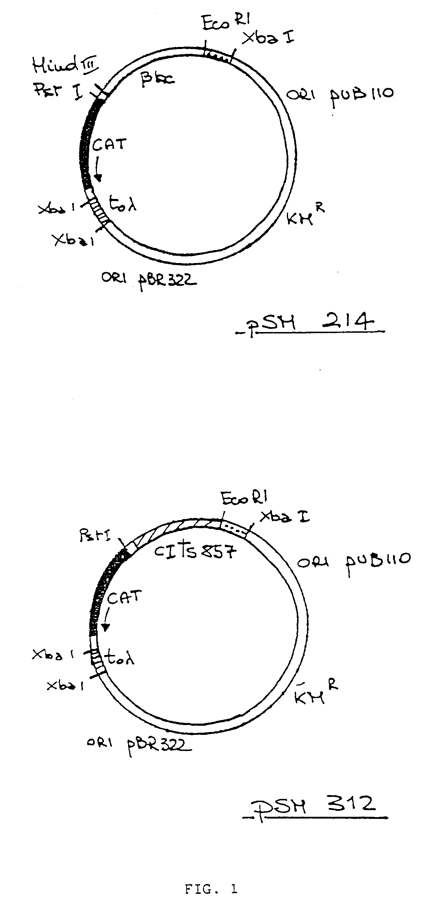

The transformants were selected on LB agar plates (DIFCO) containing 20μg/ml of chloramphenicol (Cm). The 7230 bp plasmid pSM 312 was isolated from one of the positive chloramphenicol-resistant (CmR) colonies and, upon restriction analysis, showed the correct insertion of the 710b fragment (Figure 1). One of the positive clones was designated E.coli SMC 239.

b) Introduction of pSM 312 into B.subtilis.

1μg of pSM 312 was used to transform B.subtilis SMS 118 cells (Leu, pyRD1, npr⁻, spr⁻) made competent as described by Contente and Dubnau (Mol, Gen. Genet., 167, 251-258, 1979). The transformants were selected on VY agar plates (DIFCO) containing 5μg/ml of Cm. The DNA of the positive (CmR) colonies was analysed by cutting with restriction enzymes and analysis on agarose gel, showing a pattern identical to that of pSM 312. One of the positive clones was designated B.subtilis SMS 248.

Example 3

Expression of the repressor protein in B.subtilis SMS 248 and in E.coli SMC 239.

a) B.subtilis SMS 248 (pSM 312) were grown in 10ml of VY medium with the addition of 5μg/ml of chloramphenicol at 42°C for 20 hours. 1ml of the culture was then centrifuged (Eppendorf centrifuge mod. 5450) at 12,000 rpm for 5 minutes and the precipitate was suspended in 100μl of a buffer containing 30mM pH7.2 Tris-HCl and 15% of sucrose. 20μl of a lysozyme solution (5mg/ml in 250mM pH7.2 Tris-Hcl) was then added to the mixture. The mixture was incubated at 37°C for 10 minutes to encourage cell lysis and 180 μl of a solution containing 125mM pH6.8 Tris-HCl, 20% glycerine, 3% sodium dodecyl sulphate (SDS), and 3% beta mercaptoethanol were then added.

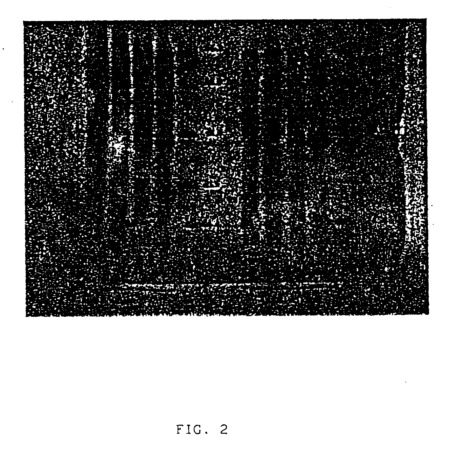

A 15μl portion, corresponding to 50μl of the initial culture, was loaded on to 12.5% SDS polyacrylamide gel (Laemmli, Nature, vol. 277, 680, 1970). After electrophoresis at 25mA for approximately 3 hours, the proteins were displayed by dyeing with Coomassie Blue (0.2% R-250 BioRad in 30% ethanol and 10% acetic acid) (Gel Electrophoresis of proteins : a practical approach, B.D. Manes, D. Rickwood, IRL Press Ltd). The dyeing of the gel revealed the presence of a proteinaceous band with a molecular weight corresponding to that of the repressor protein (Figure 2), thus confirming the ability of the microorganism to express the protein.

b) E.coli SMC 239 cells were grown in LB with the addition fo 20μg/ml of chloramphenicol at 42°C. The cells were then separated and lysed as described above and the proteinaceous lysate was analysed as described in step a). The results obtained showed that this strain was also able to express the repressor at considerable levels but less than those obtained from B.subtilis.

Example 4

Construction of the plasmid pSM 3120$_L$.

In order to evaluate whether the ts operator/repressor system of the lambda phage was also able to function in B.subtilis, the 60bp DNA fragment corresponding to the left-hand operator/promoter sequence (O$_L$/P$_L$) of the lambda phage containing the two repressor attachment sites O$_{L1}$ and O$_{L2}$ was inserted suitably in the plasmid pSM 312.

a) Introduction of the sequence O$_L$/P$_L$ into pSM 312.

1μg of pSM 312 was digested with 2U of the restriction enzymes EcoRI and SstI at 37°C for 1 hour. After a check to ensure that digestion had taken place by the loading of a portion on to 0.8% agarose gel, the DNA was extracted with equal volumes of phenol-chloroform and chloroform-isoamyl alcohol, separated by centrifuging and dried under vacuum.

Simultaneously, a One Plus (Beckmann) DNA Synthesizer was used to synthesize the following 65-base and 56-base oligonucleotides:

$O_{LA}$ (SEQ. ID. No. 3):                                    $O_{L1}$

5'A A T T C C T C C T T T G A G C T C C T A T C A

C C G C C A G T G G T A T T T A T G T C A A C A C

       $O_{L2}$

C G C C A G A G A T A G A G C T 3'


$O_{LB}$ (SEQ. ID. No. 4):

3'G G A G G A A A C T C G A G A T A G T G G C G G

T C A C C A T A A A T A C A G T T G T G G C G G T

C T C T A T C 5'


These oligonucleotides (linkers) correspond to the two helices of the $O_L/P_L$ sequence of the lambda phage containing the $O_{L1}$ and $O_{L2}$ sites suitable for binding with the repressor.

$0.5\mu g$ of the linkers were phosphorylated in $50\mu l$ of a solution containing 50mM pH7.6 Tris-HCl, 10mM $MgCl_2$, 5mM DTT, 0.1mM EDTA, 1mM ATP, 0.1mM spermidine and 10U polynucleotide kinase (Boehringer) at 37°C for 1 hour. After the kinase had been deactivated at 65°C for 10 minutes, the two solutions were mixed and incubated at ambient temperature (20-25°C) for 30 minutes to encourage coupling (annealing) of the two complementary helices.

0.1 gamma ($2\mu l$) of the plasmid pSM 312, digested as described above, and 10ng ($1\mu l$) of the double-helix linker, molar ratio 1:10, were then ligated in a ligation mixture in the presence of 1U of T4 DNA ligase at 14°C for 16 hours and then deactivated at 65°C for 10 minutes.

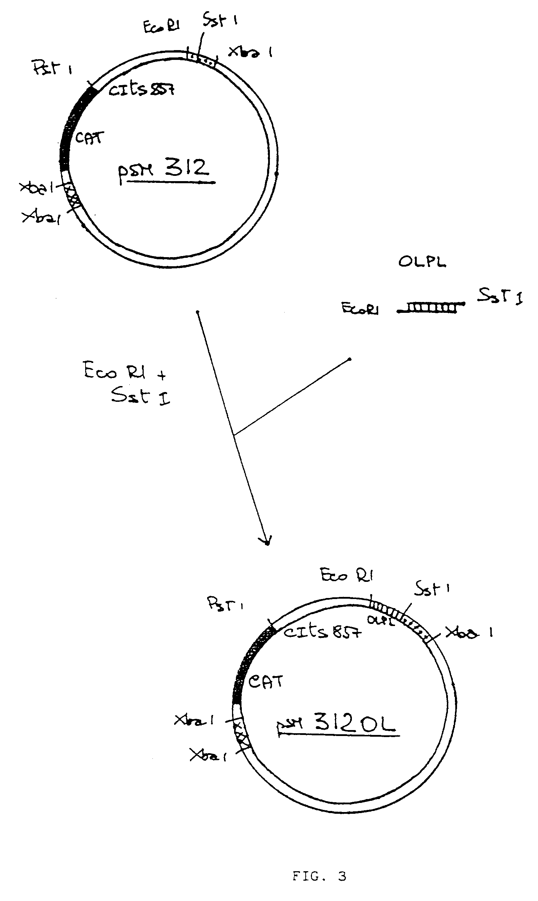

The ligation mixture thus obtained was used to transform competent E.coli 71.18 cells. The transformants were then selected on LB agar plates with the addition of $20\mu g/ml$ of Cm. Only after restriction analysis with EcoRI and XbaI enzymes could it be confirmed that the 60bp fragment had been inserted as desired in some clones. In fact, by analysis on 7% polyacrylamide gel, a 135bp band corresponding to the promoter of pSM 214 (75bp) plus $O_L/P_L$ was observed. The plasmid pSM $312O_L$ containing the desired sequence was isolated from one of the positive clones ($CM^R$), designated E.coli SMC 242 (Figure 3).

b) Insertion of pSM $312O_L$ in B.subtilis

Competent B.subtilis SMS 118 cells were transformed by the plasmid pSM $312O_L$. The clone SMS 258 (pSM $312O_L$) was isolated from the transformants selected at 37°C on VY plates containing $5\mu g/ml$ of Cm and, after restriction analysis, its plasmid DNA was found to be identical to the expected pattern.


Example 5


a) GROWTH DATA

The following Table I gives the growth data of the E.coli and B.subtilis strains transformed by the plasmids pSM 214, pSM 312 and pSM $312O_L$. These data were obtained by growth on plates of the best medium (LB per E.coli and TBAB for B.subtilis) containing kanamycin ($15\mu g/ml$ for E.coli and $5\mu g/ml$ for B.subtilis), or chloramphenicol ($20\mu g/ml$ and $5\mu g/ml$ respectively). The same strain was applied to two identical plates and these were incubated simultaneously, one at 28°C and the other at 42°C. The control strains, that is, those containing pSM 214 and pSM 312, grew well at both temperatures with both of the antibiotics. The strains containing the plasmid pSM $312O_L$, that is, those containing in which the operator/repressor system, showed differing growths at the two temperatures and on the two antibiotics. The same results were observed for the same strains grown in liquid media.

7

Table I

| Temperature | | E.coli | | B.subtilis | |
|---|---|---|---|---|---|
| | | KM | CM | KM | CM |
| pSM 214 | 28°C | + | + | + | + |
| | 42°C | + | + | + | + |
| pSM 312 | 28°C | + | + | + | + |
| | 42°C | + | + | + | + |
| pSM 312O$_L$ | 28°C | + | - | + | - |
| | 42°C | + | + | + | + |

b) Expression of the repressor protein in SMC 242 and SMS 258 (pSM 312O$_L$)

In order to evaluate the effectiveness of the expression of the total protein content for the said strains, raw proteinaceous extracts were prepared as described in Example 2 above.

The data obtained from SDS-polyacrylamide gel, given in Figure 2, show that the strains are able to express the repressor.

Example 6

Measurement of CAT activity

In order to confirm the data given in Table I and to evaluate the extent to which the expression of the gene was modulated by the ts O$_L$P$_L$/repressor system, both in E. coli and in B.subtilis, the activity was measured of chloramphenicol acetyl transferase (CAT), whose structural gene is located immediately downstream of the promoter of pSM 214 and of the O$_L$/P$_L$ system and of the lambda-phage repressor. A culture of the two strains (SMC 242 and SMS 258) was incubated at 28°C for 16 hours in a suitable medium. After this period of time, each culture was diluted by 1:100 and divided into a further two cultures, one of which was incubated at 28°C and the other at 42°C. 1ml samples were taken from the two cultures at comparable ODs. After centrifuging, the cell pellets were washed with STE (0.1M NaCl, 10mM pH8.0 Tris-HCl, 1mM EDTA) and then resuspended in 1ml 100mM of pH7.8 Tris-HCl. The solutions were then sonicated in order to lyse the cells and make the cell extract less viscous. After 4 sonication cycles for E.coli and 6 for B.subtilis, the CAT detection test was carried out. In practice, 100$\mu$l of cell extract and 1ml of the enzymatic reaction mixture were added to a dish kept thermostatically at 37°C as described by Shaw, Methods in Enzymology, 40, 737, 1974.

Controls were carried out by the testing of samples in the absence of CM. It was thus possible to measure the base CAT-like activity to be subtracted from the value obtained in the presence of CM. In all cases, this activity was zero or little more than 0, that is, negligible. The net increase in absorbance per minute was divided by 13.6 to give the result in micromoles/minute of CM-dependent DTNB.

Since this is equivalent to the acetylation value and since 1 CAT unit = 1 micromole of acetylated Cma, the value obtained immediately gives the value of the units of enzyme in the dish (absorption difference/minute/13.6 x 10 = units/ml).

The results of some enzymatic tests obtained from the cell extracts of B.subtilis are given in Table II.

## Table II

| B.subtilis SMS 118 | | | 28°C U/ml | 28°C U/OD | 42°C U/ml | 42°C U/OD |
|---|---|---|---|---|---|---|
| pSM 214 | O.D. | 0.4 | 0.058 | 0.145 | 0.065 | 0.162 |
| | " | 1.0 | 0.280 | 0.280 | 0.350 | 0.350 |
| | " | 2.3 | 0.368 | 0.160 | 0.410 | 0.178 |
| pSM 312 | O.D. | 0.4 | 0.065 | 0.162 | 0.068 | 0.170 |
| | " | 1.0 | 0.342 | 0.342 | 0.370 | 0.370 |
| | " | 2.3 | 0.397 | 0.172 | 0.430 | 0.187 |
| pSM 312O$_L$ | O.D. | 0.4 | 0.018 | 0.045 | 0.055 | 0.137 |
| | " | 1.0 | 0.093 | 0.093 | 0.290 | 0.290 |
| | " | 2.3 | 0.120 | 0.052 | 0.290 | 0.126 |

The values given are expressed both in units/ml and in units/OD. The greater activity at 42°C than at 28°C can be seen from these data.

Moreover, it should be noted that a minimum of CAT activity was measured for pSM 312O$_L$ at 28°C due to the fact that the messenger which transcribes the repressor and the CAT gene, which are present as the first and second genes of the dicystronic operon, are the same. This activity would be zero if the two genes were transcribed by two different messengers.

SEQUENCE LISTING

SEQ ID NO: 1
SEQUENCE TYPE: Nucleotide
SEQUENCE LENGHT: 31 base pairs
STRANDEDNESS: Single
TOPOLOGY: Linear
MOLECULE TYPE: Synthetic
FEATURES: From 3 to 9 EcoRI site

GTGAATTCTT  ATGAGCACAA  AAAAGAAACC  A                    31

*** ** ***

SEQ ID NO: 2
SEQUENCE TYPE: Nucleotide
SEQUENCE LENGHT: 29 base pairs
STRANDEDNESS: Single
TOPOLOGY: Linear
MOLECULE TYPE: Synthetic

GTAAGCTTTC  AGCCAAACGT  CTCTTCAGG                         29

*** ** ***

SEQ ID NO: 3
SEQUENCE TYPE: Nucleotide
SEQUENCE LENGHT: 65 base pairs
STRANDEDNESS: Single
TOPOLOGY: Linear
MOLECULE TYPE: Synthetic
IMMEDIATE EXPERIMENTAL SOURCE: (...)
FEATURES: From 1 to 4 EcoRI sticky end; from 19 to 36 $O_{L1}$
operator; from 44 to 60 $O_{L2}$ operator.

AATTCCTCCT  TTGAGCTCCT  ATCACCGCCA  GTGGTATTTA           40
TGTCAACACC  GCCAGAGATA  GAGCT                            65

*** ** ***

SEQ ID NO: 4
SEQUENCE TYPE: Nucleotide
SEQUENCE LENGHT: 56 base pairs
STRANDEDNESS: Single
TOPOLOGY: Linear
MOLECULE TYPE: Synthetic
FEATURES:  From 1 to 56 complementary to fragment 61-5 of
sequence ID NO. 3

CTATCTCTGG  CGGTGTTGAC  ATAAATACCA  TCGGCGGTGA           40
TAGAGCTCAA  AGGAGG                                       56

**Claims**

1.  A plasmid vector which can replicate in <u>Bacillus</u> <u>subtilis</u> for the inducible expression in <u>Bacillus</u> <u>subtilis</u> of a heterologous structural gene, which codes for a protein of interest, wherein said vector comprises:

- a strong promoter;
- an operator/promoter $O_L/P_L$;
- an E. coli lambda phage structural gene clts 857 encoding the heat-sensitive repressor protein of said phage;
- said heterologous structural gene;

wherein said clts 857 gene and said heterologous structural gene are under the control of said strong promoter whose activity is modulated by said operator-repressor by means of a variation in temperature.

2. A plasmid vector according to claim 1, wherein said operator/promoter $O_L/P_L$ has the following sequence:

$O_{LA}$ (SEQ. ID. No. 3):                                          $O_{L1}$

5'A A T T C C T C C T T T G A G C T C C T A T C A

C C G C C A G T G G T A T T T A T G T C A A C A C

C G C C A G A G A T A G A G C T 3'

$O_{L2}$

$O_{LB}$ (SEQ. ID. No. 4):

3'G G A G G A A A C T C G A G A T A G T G G C G G

T C A C C A T A A A T A C A G T T G T G G C G G T

C T C T A T C 5'

3. A plasmid vector according to Claim 1, in which the strong promoter is isolated from the plasmid pSM 214 ATCC 67320.

4. A plasmid vector according to Claim 3, obtainable from Bacillus subtilis ATCC 68058.

5. A microorganism selected from the Bacillus subtilis group including a plasmid vector according to Claims 1 to 4.

6. Bacillus subtilis SMS 118 ATCC 68058.

7. A method for the thermo-inducible expression of a heterologous structural gene which codes for a protein of interest, comprising the growth in a culture medium containing sources of carbon, nitrogenous sources and mineral salts, of a strain of Bacillus subtilis transformed by a plasmid vector according to Claims 1 to 4, in which the regulation of the expression is modulated by temperature variation.

**Patentansprüche**

1. Ein Plasmidvektor, der sich in Bacillus subtilis replizieren kann, für die induzierbare Expression in Bacillus subtilis eines heterologen Struktur-Gens, welches für ein interessierendes Protein codiert, wobei besagter Vektor die folgenden Komponenten umfaßt:
- einen starken Promotor
- einen Operator/Promotor $O_L/P_L$;
- ein E.coli LambdaPhage Struktur-Gen clts 857, welches für das hitzeempfindliche Repressor-Protein besagter Phage codiert;
- besagtes heterologe Struktur-Gen;

wobei besagtes clts 857 Gen und besagtes heterologe Struktur-Gen von besagtem starken Promotor kontrolliert werden, dessen Aktivität durch besagten Operator-Repressor mittels Veränderungen in der Temperatur moduliert wird.

2. Ein Plasmidvektor gemäß Anspruch 1, wobei besagter Operator/Promotor $O_L/P_L$ die nachstehende Sequenz aufweist:

```
O
 LA  (SEQ. ID. No. 3):                        O
                                               L1
5'A A T T C C T C C T T T G A G C T C C T A T C A

C C G C C A G T G G T A T T T A T G T C A A C A C

C G C C A G A G A T A G A G C T 3'

        O
         L2
O
 LB  (SEQ. ID. No. 4):
3'G G A G G A A A C T C G A G A T A G T G G C G G

T C A C C A T A A A T A C A G T T G T G G C G G T

C T C T A T C 5'
```

3. Ein Plasmidvektor gemäß Anspruch 1, wobei besagter starker Promotor aus dem Plasmid pSM 214 ATCC 67320 isoliert worden ist

4. Ein Plasmidvektor gemäß Anspruch 3, erhältlich aus Bacillus subtilis ATCC 68058.

5. Ein Mikroorganismus , ausgewählt aus der Gruppe von Bacillus subtilis einschließlich einem Plasmidvektor gemäß den Ansprüchen 1 bis 4.

6. Bacillus subtilis SMS 118 ATCC 68058.

7. Ein Verfahren zur thermisch induzierbaren Expression eines heterologen Struktur-Gens, welches für ein interessierendes Protein codiert, umfassend das Züchten eines Stammes von Bacillus subtilis in einem Kulturmedium, welches Quellen für Kohlenstoff und Stickstoff sowie Mineralsalze enthält, wobei besagter Stamm mittels eines Plasmidvektors gemäß den Ansprüchen 1 bis 4 transformiert worden ist und die Regelung der Expression durch Veränderungen in der Temperatur moduliert wird.

**Revendications**

1. Vecteur du type plasmide apte à la réplication dans Bacillus subtilis, pour l'expression inductible dans Bacillus subtilis d'un gène structural hétérologue, qui code pour une protéine intéressante, ledit vecteur comprenant :
   - un promoteur fort ;
   - un opérateur/promoteur $O_L/P_L$ ;
   - un gène structural clts 857 de phage lambda de E. coli, codant pour la protéine de répression thermosensible dudit phage ;
   - ledit gène structural hétérologue ;
   dans lequel ledit gène clts 857 et ledit gène structural hétérologue sont sous le contrôle dudit promoteur fort dont l'activité est modulée par ledit opérateur-répresseur au moyen d'une variation de température.

2. Vecteur du type plasmide suivant la revendication 1, dans lequel l'opérateur/promoteur $O_L/P_L$ possède la séquence suivante :

O$_{LA}$ (SEQ. ID. N° 3) :

O$_{L1}$

5'A A T T C C T C C T T T G A G C T C <u>C T A T C A</u>

<u>C C G C C A G T G G T A</u> T T T A T G T <u>C A A C A C</u>

<u>C G C C A G A G A T A</u> G A G C T 3'

O$_{L2}$

O$_{LB}$ (SEQ. ID. N°. 4) :

3' G G A G G A A A C T C G A G A T A G T G G C G G

T C A C C A T A A A T A C A G T T G T G G C G G T

C T C T A T C 5'

3. Vecteur du type plasmide suivant la revendication 1, dans lequel le promoteur fort est isolé du plasmide pSM 214 ATCC 67320.

4. Vecteur du type plasmide suivant la revendication 3, pouvant être obtenu à partir de <u>Bacillus subtilis</u> ATCC 68058.

5. Micro-organisme choisi dans le groupe de <u>Bacillus subtilis</u>, comprenant un vecteur du type plasmide suivant les revendications 1 à 4.

6. <u>Bacillus subtilis</u> SMS 118 ATCC 68058.

7. Procédé pour l'expression thermo-inductible d'un gène structural hétérologue qui code pour une protéine intéressante, comprenant la croissance dans un milieu de culture contenant des sources de carbone, des sources d'azote et des sels minéraux d'une souche de <u>Bacillus subtilis</u> transformée avec un vecteur du type suivant les revendications 1 à 4, dans lequel la régulation de l'expression est modulée par une variation de température.

pSH 214

pSH 312

FIG. 1

FIG. 2

FIG. 3

(A)

(B)

FIG. 4